# EUROPÄISCHE PATENTSCHRIFT

(11) **EP 2 700 394 B1**
(45) Veröffentlichungstag und Bekanntmachung des Hinweises auf die Patenterteilung: **09.09.2020**
(21) Anmeldenummer: 13003271.7
(22) Anmeldetag: 27.06.2013
(51) Int. Cl.: A61K 6/30, A61K 6/887

(54) **Verwendung eines lösemittelfreien Haftvermittlers zur Herstellung eines Haftverbunds zwischen Dentalkompositen und der Oberfläche von auf der Basis von Hochleistungspolymeren hergestellter Dentalgerüste**
Use of a solvent-free adhesion promoter to create an adhesive bond between dental composites and the surface of dental frameworks made of high-performance polymers
Utilisation d'un promoteur d'adhésion sans solvant pour créer une liason adhésive entre les composites dentaires et la surface des armatures dentaires à base de polymères haute performance

(30) Priorität: 21.08.2012 DE 102012016418
(43) Veröffentlichungstag der Anmeldung: 26.02.2014
(73) Patentinhaber: Kulzer GmbH, 63450 Hanau (DE)
(72) Erfinder: Ruppert, Klaus, 63477 Maintal (DE); Hohmann, Alfred, 61389 Schmitten (DE)
(74) Vertreter: Bendele, Tanja

(56) Entgegenhaltungen:
- DE-A1-102007 013 285
- DE-C1- 4 233 886

## Beschreibung

Die Erfindung betrifft die Haftvermittlung und die Herstellung eines Haftverbundes zwischen Hochleistungspolymeren und Dentalkompositen, insbesondere mit dentalen Verblendkompositen.

### Technischer Hintergrund:

Prothetische Arbeiten wie z.B. Kronen oder Brücken erfordern in vielen Fällen zur Gewährleistung der mechanischen Stabilität eine tragende Unterkonstruktion. Typischerweise bestehen diese Gerüste aus Metalllegierungen oder aus metallfreien Werkstoffen wie z.B. ZrO₂, Al₂O₃ oder Hochleistungspolymeren wie PEEK. Um der Zahnrestauration auf derartigen Gerüstkonstruktionen ein ästhetisches Aussehen zu verleihen, werden diese mit einem Dental-Komposit oder im Fall von Metallgerüsten einer Keramik zahnfarben verblendet. Zur Gewährleistung eines dauerhaften Verbunds wird typischerweise ein Haftvermittler verwendet.

Zunächst wird die Oberfläche mechanisch vorbehandelt, in der Regel durch Sandstrahlen. Auf das sandgestrahlte Gerüst wird zunächst eine Haftvermittlerschicht aufgebracht, welche einen Verbund zwischen der Gerüstoberfläche und den nachfolgenden (Meth)acrylat-basierten Schichten herstellt. Auf den Haftvermittler wird anschließend eine Schicht eines relativ transparenten, fließfähigen Materials aufgebracht, welches auch in die Toträume in den Hinterschneidungen einfließen und diese auffüllen kann. Dabei muss sichergestellt sein, dass das Material dort ausreichend aushärtet. Auf diese fließfähige Schicht können anschließend eine oder mehrere Schichten Opaker aufgebracht werden, um das Gerüst farblich zu kaschieren. Den Abschluss des Schichtausbaus bildet dann das eigentliche Verblendkomposit.

### Stand der Technik

WO2008/113541A2 betrifft Konditionierungsmittel, die für Polymere ausgewählt aus Polyarylaten, Polyarylensulfiden, Polysulfonen, flüssigkristallinem Polymer, Polyimiden, Polyetherimiden, Polyamidimiden, Polyaryletherketonen, geeignet sind. Die Konditionierungsmittel enthalten ein Haftmittel und ein höhersiedendes Lösemittel mit Dipolcharakter, ausgewählt aus Dimethylsulfoxid, Phenol, Diphenylsulfon, Cylohexanon, Acetylaceton und Ethylenglykol. Die Anwendung umfasst Auftragen des Konditionierungsmittels auf zumindest einen Teil der Oberfläche des Formkörpers, Einwirkenlassen des Konditionierungsmittels, und optional Auftragen einer härtbaren Mischung auf die mit Konditionierungsmittel konditionierte Oberfläche des Formkörpers. Die Beispiele betreffen DMSO-haltige Konditionierungsmittel. DMSO ist gemäß Sicherheitsdatenblatt nicht unbedenklich und nicht für die Anwendung im Mund geeignet (Produktname Dimethylsulfoxide (DMSO).

DE4233886C1 betrifft ein polymerisierbares Konditionierungsmittel auf Methacryl-Basis und ein Verfahren zur Vorbehandlung der Oberfläche von Formkörpern aus Polyacrylat-, Polymethacrylat- und Polycarbonat-Kunststoffen vor dem Auftragen von polymerisierbarem Methacrylat-Material.

### Aufgabenstellung:

Es ist ein Verfahren anzugeben, mit dem ein dauerhafter Verbund zwischen einem Hochleistungspolymer wie z.B. PEEK und einem (meth-)acrylatbasierten Verblendkomposit hergestellt werden kann. Eventuell als problematisch angesehene Lösungsmittel sollten vermieden werden.

### Erfindung:

Die Aufgabe wird durch ein Verfahren nach Anspruch 1 gelöst. Vorteilhafte Ausgestaltungen sind den weiteren Ansprüchen zu entnehmen.

Insbesondere wird die Oberfläche des Gerüstes mechanisch vorbehandelt, vorzugsweise sandgestrahlt, um die Oberfläche zu reinigen, zu aktivieren und retentive Verankerungen zu schaffen. Das Strahlmittel ist bevorzugt 110 µm Korund.

Anschließend erfolgt der Auftrag einer lichthärtenden, gering viskosen oder fließfähigen, lösemittelfreien Methacrylat-basierten Komponente (Haftvermittler bzw. Konditionierungsmittel), welche einen dünnen Film auf der Gerüstoberfläche bildet und nach Härtung zusätzlich zu einem retentiven Haftverbund führt. Sie enthält, PMMA (Polymethylmethacrylat), MMA (Methylmethacrylat), bifunktionelle Methacrylat-basierte Monomere wie z.B. UDMA (Urethandimethacrylat), und Photoinitiator(en) sowie optional Stabilisatoren und anorganische Pigmente. Ferner können ebenfalls optional Mittel zur Einstellung der Viskosität vorhanden sein, wie etwa feinteilige Kieselsäuren, die in dem Fachmann bekannter Weise silanisiert sein können.

Die allgemeine Rezeptur eines derartigen Haftvermittlers ist beispielsweise wie folgt (in Gew.%):

| | |
|---|---|
| 60 bis 70 % | MMA |
| 5 bis 15% | PMMA |
| 20 bis 30 % | UDMA |
| 0,1 bis 5 % | Photoinitiator, vorzugsweise aus der Gruppe der Acylphosphin-Photoinitiatoren, |
| 0,05 bis 5 % | Stabilisator, bevorzugt aus der Gruppe der sterisch gehinderten Phenole, wie z.B. 2,6-ditert.-butyl-4-methyl phenol. |

Das Polymethylmethacrylat kann bei der Zubereitung des Haftvermittlers als solches, vorzugsweise in Form von Polymerisat-Perlen mit einer Teilchengröße von 10-150 Mikrometer, oder als Lösung (oder teilweise Lösung) in den Methylmethacrylat-Monomeren, eingesetzt werden. Ein Polymethylmethacrylat mit einem mittleren Molekulargewicht von 120 000 - 200 000 wird dabei bevorzugt.

Bei der aufgebrachten Haftschicht hat sich eine Schichtdicke von 0,5 bis 2,5 µm bei einfachem Auftragen und von 4,5 bis 7,5 µm bei zweifachem Auftragen des Haftvermittlers als vorteilhaft erwiesen. Die Mitte dieser Bereiche ist besonders bevorzugt, also um 1-2 µm bei einfachem Auftragen und um 5 µm bei zweifachem Auftragen. Bei dreifachem Auftragen werden Schichtdicken um 10 µm erreicht

Die Viskosität des Haftvermittlers liegt vorzugsweise im Bereich von ca. 10 - 30 mPa·s bei 23 °C. Als Mittel zur Einstellung der Viskosität kommen neben der Wahl der eingesetzten Monomere, Oligomere und Polymere auch Zusatzstoffe in Frage, z.B. Kieselsäuren, davon in erster Linie pyrogene Kieselsäuren. Das Gerüst, z.B. auf PEEK-Basis, kann nach Behandlung mit dem Haftvermittler z.B. mit einem Verblendkomposit verblendet werden. Beispiele sind Signum® composite oder Signum® matrix der Firma Heraeus Kulzer.

Die Aushärtung erfolgt bevorzugt durch einen Härtemechanismus im UV oder sichtbaren Spektralbereich. Auch der Einsatz neuartiger IR sensitiver Initiatoren ist möglich.

Weiterer Vorteil des erfindungsgemäßen Verfahrens ist eine einfache Verarbeitung und der Verzicht auf gefährliche und/oder toxische Chemikalien (wie z. B. in WO2008/113541A2 vorgeschlagen). Außerdem ist keine Einwirkzeit der Beschichtung notwendig.

Als Dentalkomposite kommen die üblichen Dental-Kompositmischungen auf der Basis von Monomermischungen und Füllstoffen in Frage.

Beispiele geeigneter Monomere sind die im Dentalbereich üblichen, wie z.B. monomere (Meth)acrylate wie Ethylenglycoldimethacrylat EDMA, Diethylenglycoldimethacrylat, Triethylenglycoldimethacrylat TEGDMA, Glyceroldimethacrylat GDMA, Glyceroltrimethacrylat, Trimethylolpropantrimethacrylat, Pentaerythritoldimethacrylat, Pentaerythritoltrimethacrylat, Pentaerythritoltetramethacrylat, Derivate von Bisphenol A wie Bisphenol-A-dimethacrylat und Bisphenol-A-diglycoldimethacrylat, Urethanmethacrylat erhältlich aus Diisocyanaten und Hydroxyalkylmethacrylaten, sowie Reaktionsprodukte von Polyolen, Diisocyanaten und Hydroxyalkylmethacrylaten gemäß DE3703080A1 oder DE3703120A1; C₁₋₁₂-, vorzugsweise C₁₋₄-Alkylmethacrylate wie Methylmethacrylat, Ethylmethacrylat, n-Propylmethacrylat, Isopropylmethacrylat, n-Butylmethacrylat and t-Butylmethacrylat, Hydroxyalkyl-C₁₋₄-methacrylate wie 2-Hydroxyethylmethacrylat, 2-Hydroxypropylmethacrylat, Diethylenglycolmonomethacrylat, Triethylenglycolmonomethacrylat, Alkoxy-C₁₋₄ alkylmethacrylate wie 2-Methoxyethylmethacrylat, 3-Methoxybutylmethacrylat und Ethyltriglycolmethacrylat. Geeignete Monomere sind davon jeweils die Monomere selbst, daraus hergestellte polymerisierbare Präpolymere sowie Mischungen von diesen.

Geeignete Füllstoffe sind dem Fachmann bekannt. Beispiele sind inerte und reaktive Dentalgläser wie Bariumsilikatglas, Strontiumsilikatglas, Borosilikatglas und Fluoralumosilikatglas oder pyrogene, gefällte oder fossile Kieselsäuren. Bevorzugt werden Mischungen verschieden großer Füllstoffpartikel mit Teilchengrößen zwischen 0,001 und 100 µm eingesetzt.

Weiter sind geeignet mikro- und/oder nanoskalige Füllstoffe, z.B. Metall-, Halbmetall- oder Mischmetalloxide, Silikate, Nitride, Sulfate, Titanate, Zirkonate, Stannate, Wolframate, Siliciumdioxid oder Mischungen aus diesen Verbindungen, ferner sphärische Füllstoffe, Pulver weiterer Gläser oder Glaskeramiken und deren Mischungen, sowie außerdem gefüllte oder ungefüllte Splitterpolymerisate und/oder Perlpolymerisate.

Die Füllstoffe können oberflächenmodifiziert, vorzugsweise organisch oberflächenmodifiziert wie beispielsweise silanisiert sein. Ein oberflächenmodifizierter Füllstoff kann auf seiner Oberfläche funktionelle Gruppen besitzen, die mit den Monomeren chemisch, vorzugsweise radikalisch, reagieren können oder eine hohe Affinität zu der aus den Monomeren gebildeten Polymermatrix haben, wobei der Füllstoff vorzugsweise mit reaktive Acrylat- oder Methacrylatgruppen tragendem Silan oberflächenmodifiziert ist.

Das erfindungsgemäße Verfahren dient vorzugsweise der Haftvermittlung zwischen dem Gerüst aus Hochleistungspolymer und den auf die Haftvermittlerschicht aufgebrachten Schichten sogenannter Opaker (das sind stark weißpigmentierte Composite zum Kaschieren der Gerüstfarben). Es können aber auch direkt Dentalkomposite aufgetragen werden.

Das Verfahren nach der Erfindung zeichnet sich durch seine Einfachheit und Effizienz aus: Auftragen, Belichten, kein langes Warten auf Eindringen, die nächste Schicht (z.B. Opaker Dental-komposit) kann sofort aufgetragen werden, und es ergibt sich ein fester und dauerhafter Verbund.

Als Hochleistungspolymere kommen in Frage: Polyetheretherketon (PEEK), Polyetherketonketon (PEKK), Polyoxymethylen (POM), sowie Polyamide wie z.B. die in den Beispielen eingehend beschriebenen Zellamid® Typen.

Die folgenden Beispiele zeigen, dass die Haftwerte am besten sind, wenn zweimal mit dem Haftvermittler nach der obigen allgemeinen Rezeptur behandelt wird. Ein dreimaliges oder öfteres Aufbringen ist ebenfalls möglich. Die Beispiele dienen der näheren Erläuterung der Erfindung. Teile- und Prozentangaben beziehen sich wie in der übrigen Beschreibung auf das Gewicht, sofern nicht anders angegeben.

### Ausführungsbeispiele:

Auf den Gerüstwerkstoff wird nach dem Sandstrahlen mit 110 µm Korund und dem Abspülen mit voll entsalztem Wasser und dem Trocknen mit ölfreier Luft der Haftvermittler 2 x aufgetragen und mit der Laborlichtlampe Heraflash® (Fa. Heraeus Kulzer) für 90 sec. belichtet. Anschließend werden 2 Schichten Signum® opaque F (Fa. Heraeus Kulzer) aufgetragen und jeweils für 90 sec. mit Heraflash® ausgehärtet. Den Abschluss bildet eine Schicht aus Signum® composite (Fa. Heraeus Kulzer), evtl. in verschiedenen Farben, die mit 90 sec. zwischengehärtet und anschließend zur Vergütung nochmals 180 sec. endgehärtet werden.

Mit diesem Vorgehen hergestellte Prüfkörper ergeben folgende Messwerte für die Prüfung des Scherhaftverbundes nach ISO 10477 (Tabelle A):

| Gerüstwerkstoff | Vestkeep / Evonik | | |
|---|---|---|---|
| Haftvermittler | x | x | 1x |
| Signum opaque F | x | 2x | 2x |
| Signum composite | JA | | |
| TWL (5 °C/55 °C) | 5000 | | |
| Scherhaftverbund [MPa] | 2,6 | 14,5 | 18,5 |

Nach ISO 10477 muss ein Scherhaftverbund von mind. 5 MPa erreicht werden, wenn man ohne makromechanische Retentionen arbeitet. Nach ISO 10477 werden die Prüfkörper vor dem Abscheren zuvor einer Temperaturwechsellast von 5000 Zyklen unterzogen.

Überraschenderweise kommt es zu einer mehr als zufriedenstellenden Haftung mit dem erfindungsgemäßen Haftvermittler, die deutlich über den Normerfordernissen liegt.

Der erfindungsgemäße Haftvermittler ist auch für andere Hochleistungspolymere außer PEEK geeignet. Dies ist der folgenden Tabelle B zu entnehmen: Es zeigt sich, dass der Haftverbund bei unterschiedlichen Polymeren sehr deutlich über der Norm liegt und damit mehr als ausreichend ist:

| Gerüstwerkstoff | Zellamid 202 (PAG) | Zellamid 900 (POM) | Zellamid 202 XN | Bioloren |
|---|---|---|---|---|
| Haftvermittler | 2x | 2x | 2x | 2x |
| Signum opaque F | 2x | 2x | 2x | 2x |
| Signum composite | JA | JA | JA | JA |
| TWL (5 °C/55 °C) | 5000 | 5000 | 5000 | 5000 |
| Scherhaftverbund [MPa] | 19,5 | 18,6 | 18,5 | 22,8 |

Diese Werte sind denen des Standes der Technik WO2008/113541A2 um bis zu 46 % überlegen (dort 15,2 MPa für Dentanium, ein Verbundwerkstoff auf PEEK Basis).

Die einzelnen Hochleistungspolymere in der vorstehenden Tabelle sind kommerziell erhältlich und nach Herstellerangaben wie folgt zu beschreiben:
Zellamid® 900/POM-C (Fa. Zell-Metall Ges.mbH. Engineering Plastics, Kaprun - Austria)

POM-C ist ein semikristalliner Thermoplast, hergestellt aus Acetalcopolymerisatgranulaten, und zeichnet sich durch einen niederen Reibbeiwert und gute Verschleißfestigkeit aus. Da die Wasseraufnahme sehr gering ist, ist die Dimensionsstabilität viel besser als bei Polyamiden. POM ist gegen viele Chemikalien und auch Lösungsmittel beständig. POM bietet hohe Festigkeit und Steifigkeit bei einfacher Bearbeitbarkeit.

ZELLAMID® 900 ist auch bekannt für seine hohe mechanische Festigkeit, Hitzebeständigkeit und guten Gleiteigenschaften. ZELLAMID® 900 ist nach ASTM D-61 00 frei von Mittelporosität und die meisten Qualitäten sind für den Lebensmittelkontakt zugelassen (BfR, FDA). Gut geeignet für Teile, die in feuchter oder nasser Umgebung eingesetzt werden. POM-C ist besser gegen heißes Wasser beständig als POM-H (Homopolymer).

ZELLAMID® 202(Fa. Zell-Metall Ges.mbH. Engineering Plastics, Kaprun - Austria):
ZELLAMID® 202 ist ein zähes Material mit hoher Abrieb- und Schlagfestigkeit auf der Basis von Polyamid 6 (PA 6). PA 6 wird oft als Ersatzmaterial für Bronze, Aluminium und andere Nichteisenmetalle verwendet, da es erhebliche Gewichtsvorteile bietet. So hat ZELLAMID® 202 ein spezifisches Gewicht von 1,15 g/cm³ und Bronze 8,8 g/cm³, welches ein sehr attraktives Preis-Leistungs-Verhältnis ergibt. Bei Gleitanwendung von ZELLAMID® 202 kann die Schmierung verringert werden und der Abrieb der Metallgegenfläche wird reduziert. Daher werden insgesamt sehr gute mechanische Eigenschaften geboten. PA 6 kann bis zu 8 % Wasser (gewichtsmäßig) in feuchter Umgebung oder in Wasser aufnehmen. Das erhöht die hervorragende Schlag- und Dauerbruchfestigkeit (auch bei Vibrationen) zusätzlich, kann aber auch zu Dimensionsveränderungen führen. Mechanische, elektrische Eigenschaften, wie auch die Dimensionsstabilität werden auch durch die Feuchtigkeit beeinflusst. ZELLAMID® 202 ist für den Lebensmittelkontakt zugelassen (BfR, FDA).

ZELLAMID® 202 XN (Fa. Zell-Metall Ges.mbH. Engineering Plastics, Kaprun - Austria):
Dieses Polyamid ist ein hochtechnologisches Material, das von Zell-Metall Engineering Plastics unter verwendungsneuester Technologien (Nanotechnologie) entwickelt wurde. Dieses einzigartig verstärkte PA 6 übertrifft leistungsmäßig normales PA 6, PA 6.6 und auch einige Eigenschaften von PA 6.6 mit 30 % Glasfasern. ZELLAMID® 202 XN hat eine erhöhte Dauergebrauchstemperatur von 140 °C mit einer HDT von 168 °C. Erhöhte mechanische Festigkeit mit einem E-Modul von 4200 MPa (ISO 527, trocken) sowie verringerte Wasseraufnahme, welche eine verbesserte Dimensionsstabilität sicherstellen, sind weitere hervorzuhebende Eigenschaften. Dieses Produkt ist für den Nahrungsmittelkontakt (BfR, FDA) zugelassen und bietet im Vergleich zu glasgefüllten Polyamiden ein um ca. 15 % geringeres spezifisches Gewicht, was reduzierte Volumenkosten ergibt. Der flammhemmende Effekt der Nanopartikel bringt eine Verbesserung des Brandverhaltens.

ZELLAMID 202 XN ist die Alternative für viele Anwendungen, bei denen andere Produkte die notwendigen Eigenschaften (z.B. erhöhte Gebrauchstemperatur) nicht aufweisen, da Standardmaterialien oft zu weich, wie z.B. PTFE oder zu kostspielig wie PEEK sind. Im Vergleich zu glasgefüllten Polyamiden ist dieses Material leichter zu bearbeiten, da kein Vorwärmen oder die Verwendung von Diamant-Werkzeug notwendig ist.

VESTAKEEP® PEEK Typen für die Medizintechnik (Evonik Industries AG, Essen, Deutschland):
Für Anwendungen in der Medizintechnik sind die Produkte VESTAKEEP® M2G, VESTAKEEP® 12G, VESTAKEEP® M4G, VESTAKEEP® 14G, und das Pulver VESTAKEEP® M4P erhältlich. Die Rezeptur dieser Produkte ist auf eine hohe Biokompatibilität abgestimmt und eine Chargenprüfung "in vitro" auf Zytotoxizität nach EN ISO 10993-5 bietet zusätzliche Sicherheit.

## Patentansprüche

1. Verfahren zur Herstellung eines Haftverbunds zwischen (a) der Oberfläche auf Basis von Hochleistungspolymeren umfassend Polyetheretherketon (PEEK), Polyetherketonketon (PEKK), Polyoxymethylen (POM) oder Polyamide
hergestellter dentaler Gerüste und (b) Dentalkompositen durch
A Schaffen von retentiven Verankerungen durch mechanische Vorbehandlung der Oberfläche des jeweiligen Gerüstes;
B Aufbringen eines gering viskosen oder fließfähigen, lösemittelfreien Haftvermittlers enthaltend eine Monomermischung aus Polymethylmethacrylat, Methylmethacrylat, mindestens ein bifunktionelles Methacrylat-basiertes Vernetzermonomer, Initiator(en) und optional Stabilisator(en) und sonstige Additive, mit
**(i)** Benetzung der Oberfläche,
**(ii)** Eindringen in vorhandene Vertiefungen und
**(iii)** Bildung einer Dispersionsschicht oder eines Films mit einer Schichtdicke von 0,5 bis 2,5 µm bei einfachem Auftragen und von 4,5 bis 7,5 µm bei zweifachem Auftragen des Haftvermittlers;
C zumindest teilweises Härten der Dispersionsschicht oder des Films aus Schritt B; und
D Aufbringen eines Dentalkomposits oder eines Opakers auf das Produkt des Schrittes C.

2. Verfahren nach Anspruch 1, wobei der Haftvermittler UDMA, MMA, PMMA, Photoinitiator(en) und Stabilisator(en) enthält.

3. Verfahren nach Anspruch 2, wobei das PMMA teilweise in MMA gelöst vorliegt.

4. Verfahren nach einem der vorhergehenden Ansprüche, wobei der Haftvermittler ein Mittel zur Einstellung der Viskosität enthält.

5. Verfahren nach einem der vorhergehenden Ansprüche, wobei der Haftvermittler feinteilige Fällungskieselsäure oder pyrogene Kieselsäure enthält.

6. Verfahren nach einem der vorhergehenden Ansprüche, wobei die mechanische Vorbehandlung der Oberfläche des durch Sandstrahlen erfolgt.

7. Verfahren nach einem der vorstehenden Ansprüche, wobei das Sandstrahlen mit 110 µm Korund erfolgt.

8. Verfahren nach einem der vorstehenden Ansprüche, wobei der Haftvermittler eine Viskosität von 10 - 30 mPa·s bei 23 °C aufweist.

9. Verfahren nach einem der vorstehenden Ansprüche, wobei der Haftvermittler zusätzlich anorganische Pigmente enthält.

10. Verfahren nach einem der vorstehenden Ansprüche, wobei das Aufbringen in D in mehreren Schichten erfolgt.

11. Verfahren nach einem der vorstehenden Ansprüche, bei dem zwischen C und D keine Wartezeit eingehalten wird.

12. Nicht therapeutische Verwendung eines lösemittelfreien Haftvermittlers enthaltend Initiator(en), Polymethylmethacrylat, Methylmethacrylat-Monomere und bifunktionelle Methacrylat-basierte Vernetzer,
zur Herstellung eines Haftverbunds zwischen (a) der Oberfläche auf Basis von Hochleistungspolymeren umfassend Polyetheretherketon (PEEK), Polyetherketonketon (PEKK), Polyoxymethylen (POM) oder Polyamide
hergestellter dentaler Gerüste und (b) Dentalkompositen, wobei das jeweilige Gerüst durch mechanische Vorbehandlung der Oberfläche mit retentiven Verankerungen versehen ist und die Schichtdicke des Haftvermittlers von 0,5 bis 2,5 µm bei einfachem Auftragen und von 4,5 bis 7,5 µm bei zweifachem Auftragen beträgt.

## Claims

1. Method for the production of an adhesive bond between (a) the surface of dental frameworks produced on the basis of high-performance polymers comprising polyetheretherketone (PEEK), polyetherketoneketone (PEKK), polyoxymethylene (POM) or polyamides, and (b) dental composites, by
**A** creating retentive anchorings by means of mechanical pre-treatment of the surface of the respective framework;
**B** applying a low viscous or flowable, solvent-free adhesive agent containing a monomer mixture of polymethyl methacrylate, methyl methacrylate, at least one bifunctional methacrylate-based cross-linking monomer, initiator(s), and optionally stabiliser(s) and other additives, including
**(i)** wetting of the surface,
**(ii)** penetration into existing cavities, and
**(iii)** formation of a dispersion layer or a film having a layer thickness of 0.5 to 2.5 µm, for single application, and of 4.5 to 7.5 µm, for double application, of the adhesive agent;
**C** curing the dispersion layer or the film of step B at least in part; and
**D** applying a dental composite or an opaque onto the product of step C.

2. Method according to claim 1, wherein the adhesive agent contains UDMA, MMA, PMMA, photoinitiator(s) and stabiliser(s).

3. Method according to claim 2, wherein the PMMA is partially dissolved in MMA.

4. Method according to any one of the preceding claims, wherein the adhesive agent contains an agent for adjusting the viscosity.

5. Method according to any one of the preceding claims, wherein the adhesive agent contains fine precipitated silica or pyrogenic silica.

6. Method according to any one of the preceding claims, wherein mechanical pre-treatment of the surface is carried out by sandblasting.

7. Method according to any one of the preceding claims, wherein sandblasting is carried out with 110 µm corundum.

8. Method according to any one of the preceding claims, wherein the adhesive agent has a viscosity of 10 - 30 mPa·s at 23 °C.

9. Method according to any one of the preceding claims, wherein the adhesive agent additionally contains inorganic pigments.

10. Method according to any one of the preceding claims, wherein the application in D is carried out in several layers.

11. Method according to any one of the preceding claims, wherein no waiting time is observed between C and D.

12. Non-therapeutic use of a solvent-free adhesive agent containing initiator(s), polymethyl methacrylate, methyl methacrylate monomers and bifunctional methacrylate-based cross-linking agents,
for the production of an adhesive bond between (a) the surface of dental frameworks produced on the basis of high-performance polymers comprising polyetheretherketone (PEEK), polyetherketoneketone (PEKK), polyoxymethylene (POM) or polyamides, and (b) dental composites, the respective framework being provided with retentive anchorings by mechanical pre-treatment of the surface, and the layer thickness of the adhesive agent being from 0.5 to 2.5 µm, for single application, and from 4.5 to 7.5 µm, for double application.

## Revendications

1. Procédé pour la production d'une liaison adhésive entre (a) la surface des charpentes dentaires produites à base de polymères à haute performance comprenant le polyétheréthercétone (PEEK), le polyéthercétonecétone (PEKK), le polyoxyméthylène (POM) ou des polyamides, et (b) des composites dentaires, par
**A** créer des ancrages retenants par prétraitement mécanique de la surface de la charpente respective ;
**B** appliquer un agent adhésif sans solvent de faible viscosité ou fluide contenant un mélange de monomères du polyméthacrylate de méthyle, du méthacrylate de méthyle, au moins un monomère bifonctionnel de réticulation à base de méthacrylate, des initiateur(s), et facultativement des stabilisateur(s) et des autres additifs, incluant
**(i)** mouillage de la surface,
**(ii)** pénétration dans des cavités existantes, et
**(iii)** formation d'une couche de dispersion ou d'un film ayant une épaisseur de couche de 0,5 à 2,5 µm, pour une application unique, et de 4,5 à 7,5 µm, pour une application double, de l'agent adhésif ;
**C** durcir la couche de dispersion ou le film de l'étape B au moins en partie ; et
**D** appliquer un composite dentaire ou un opaque sur le produit de l'étape C.

2. Procédé selon la revendication 1, selon lequel l'agent adhésif contient de l'UDMA, du MMA, du PMMA, des photoinitiateur(s) et des stabilisateur(s).

3. Procédé selon la revendication 2, selon lequel le PMMA est présent partiellement dissous dans le MMA.

4. Procédé selon l'une des revendications précédentes, selon lequel l'agent adhésif contient un agent pour ajuster la viscosité.

5. Procédé selon l'une des revendications précédentes, selon lequel l'agent adhésif contient de la silice de précipitation fine ou de la silice pyrogénée.

6. Procédé selon l'une des revendications précédentes, selon lequel le prétraitement mécanique de la surface est effectué par sablage.

7. Procédé selon l'une des revendications précédentes, selon lequel le sablage est effectué avec du corindon de 110 µm.

8. Procédé selon l'une des revendications précédentes, selon lequel l'agent adhésif a une viscosité de 10 - 30 mPa·s à 23 °C.

9. Procédé selon l'une des revendications précédentes, selon lequel l'agent adhésif contient en outre des pigments inorganiques.

10. Procédé selon l'une des revendications précédentes, selon lequel l'application en D est effectuée en plusieurs couches.

11. Procédé selon l'une des revendications précédentes, selon lequel il n'y a pas de temps d'attente entre C et D.

12. Utilisation non thérapeutique d'un agent adhésif sans solvent contenant des initiateur(s), du polyméthacrylate de méthyle, des monomères de méthacrylate de méthyle et des agents bifonctionnels de réticulation à base de méthacrylate
pour la production d'une liaison adhésive entre (a) la surface des charpentes dentaires produites à base de polymères à haute performance comprenant du polyétheréthercétone (PEEK), du polyéthercétonecétone (PEKK), du polyoxyméthylène (POM) ou des polyamides, et (b) des composites dentaires, la charpente respective étant fournie avec des ancrages retenants par prétraitement mécanique de la surface, et l'épaisseur de couche de l'agent adhésif étant de 0,5 à 2,5 µm, pour une application unique, et de 4,5 à 7,5 µm, pour une application double.
